(19) Europäisches Patentamt
European Patent Office
Office européen des brevets

(11) Numéro de publication: **0 179 023 B1**

(12) **FASCICULE DE BREVET EUROPEEN**

(45) Date de publication du fascicule du brevet: **23.01.91**

(21) Numéro de dépôt: **85810470.6**

(22) Date de dépôt: **15.10.85**

(51) Int. Cl.⁵: **C 08 G 69/08,** A 61 K 9/52, A 61 L 27/00

(54) **Polypeptide biodégradable et son utilisation pour le relargage progressif de médicaments.**

(30) Priorité: **19.10.84 CH 5021/84**

(43) Date de publication de la demande:
**23.04.86 Bulletin 86/17**

(45) Mention de la délivrance du brevet:
**23.01.91 Bulletin 91/04**

(84) Etats contractants désignés:
**AT BE CH DE FR GB IT LI LU NL SE**

(56) Documents cités:
**EP-A-0 105 777**
**EP-A-0 130 935**
**EP-A-0 159 293**
**US-A-4 351 337**
**US-A-4 356 166**

(73) Titulaire: **BATTELLE MEMORIAL INSTITUTE**
**7 route de Drize**
**CH-1227 Carouge/Genève (CH)**

(72) Inventeur: **Bichon, Daniel**
**Les Lieux dits Les Chenevières 16, rue de Vallard**
**F-74240 Gaillard (FR)**
Inventeur: **Lamy, Bernard**
**Chemin Jules Vuy 23**
**CH-1227 Carouge (CH)**

(74) Mandataire: **Dousse, Blasco et al**
**7, route de Drize**
**CH-1227 Carouge/Genève (CH)**

**EP 0 179 023 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention a pour objet un polypeptide estérifié biodégradable nouveau dans lequel ou peut incorporer des médicaments, ceux-ci étant, ensuite, libérés progressivement au fur et à mesure de la dégradation biochemique du polymère.

Depuis plusieurs années, on connaît des polymères biodégradables non-toxiques pouvant servir de réservoir de médicaments et permettant la libération progressive contrôlée de ceux-ci dans l'organisme lors de la dégradation du polymère porteur. On trouve des informations générales sur de tels produits dans l'ouvrage: "Fundamental Aspects od Biocompatibility" par D. F. WILLIAMS, CRC Press (1981). voir aussi brevet USP 4,093,709.

Parmi ces polymères, on cite plus particulièrement les polypeptides synthétiques (polyaminoacides) dont la structure est voisine de celle des protéines. Ces polypeptides sont biocompatibles et leurs produits de dégradation (acides aminés) sont résorbables par l'organisme. Ainsi SIDMAN et al (J. Membr. Sci (1980), 7(3), 277—91) ont divulgué un copolymère d'acide glutamique et de γ-glutamate d'éthyle dont la vitesse de dégradation est fonction de la composition du coplymère (proportions molaires des segments estérifiés par rapport aux segments non estérifiés) et que permet d'emmagasiner de nombreux produits médicamenteux, notamment des stéroïdes, des peptides, des produits anti-maleria, anti-cancer et autres. De tels polymères peuvent être utilisés sous forme de baguettes contenant, en mélange, le médicament désiré ou sous forme de capsules renfermant le médicament si celui-ci n'est pas miscible avec le polymère. Par ailleurs, les polyglutamates et polyaspartates d'alcoyle (esters simples de ces polyacides) ne sont dégradables en temps utile (d'une ordre de grandeur compatible avec leur utilisation pharmaceutique) que sous forme partiellement hydrolysée (voir par exemple ASANO et al, J. Macromol. Sci. Chem. *A21* (5) (1984), 561—582). Pour obtenir de tels polymères partiellement estérifiés, il faut soumettre ces polyglutamates ou polyaspartates à une réaction d'hydrolyse ménagée dont les conditions sont très difficilement reproductibles. Par ailleurs, de très faibles différences dans le degré d'hydrolyse influent considérablement sur la vitesse de biodégradabilite ultérieure ce qui constitue un problème additionnel dans l'emploi de tels polymères pour les buts précités.

Aussi, malgré l'intérêt que présentent les produits ci-dessus, on a continué à chercher un produit de qualités améliorées et présentant notamment les propriétés suivantes:

1. Excellente solubilité dans la plupart des solvants inoffensifs courants convenant aux médicaments (en effet, les dérivés connus de polyaminoacides ne sont, en général, solubles que dans certains solvants spéciaux (DMF, pyridine, $F_3CCOOH$) dont l'emploi est incommode pour les produits pharmaceutiques).

2. Thermoformabilité. Les polypeptides de synthèse actuellement connus ne sont en effet généralement pas miscibles avec les plastifiants biocompatibles usuels (polyacoylène glycols) et, de ce fait, ils ne sont pas thermoplastiques.

3. Contrôle amélioré du processus de dégradation. En effet, la vitesse de dégradation des polypeptides synthétiques connus est liée de façon difficilement reproductible à leur structure chimique et notamment au taux d'estérification. Ainsi, dans un cas donné (voir SIDMAN K. R., et al., PB 81—132136 NTIS (1980), p. 42) une variation du taux d'estérification de l'ordre de 10% fait passer la vitesse de dégradation de 1 au centuple (voir également la référence: SIDMAN citée plus haut).

Le polymère de l'invention a permis de réaliser ces améliorations. Il s'agit d'un polypeptide estérifié de formule:

$$\begin{array}{l} -(NH-CH-CO)_x- \\ \qquad\quad | \\ \qquad (CH_2)_n-COO-CHR^1-COOR \end{array} \qquad (I)$$

dans laquelle $R^1$ est un alcoyle inférieur ou l'hydrogène, avec R un reste aliphatique ou aromatique substitué ou non, (notamment un reste polyoxyalcoylénique hydroxylé ou alcoxylé) ou bien $R^1$ et R, liés l'un à l'autre, forment par l'intermédiaire du groupe —COO— un cycle lactonique à 5 ou 6 maillons, n vaut 1 ou 2 et x est tel que la masse moléculaire soit d'au moins 5000 D.

Comme on le voit de par la formule I, le polymère de l'invention est un polyaspartate ou polyglutamate estérifié par un ester hydroxyacétique (lactique ou glycolique) ($HO-CHR^1-COOR$) dans lequel R est, soit un reste organique monovalent soit un chaînon relié à $R^1$ de manière à former un cycle. La structure du groupe R n'est pas critique et on n'a pas rencontré de cas où, R faisant partie de composés à fonction estérifiable par les dérivés lactiques, le composant correspondant de l'invention ne puisse être obtenu. On préfère, cependant, utiliser des composés dans lequel R est un groupe aliphatique, alicyclique, aromatique ou alcoylaromatique substitué on non substitué, les substituants étant choisis parmi les fonctions organiques biocompatibles. Parmi les groupes R préférés, on peut citer les groupes méthyle, éthyle, propyle, isopropyle, isobutyle, tert-butyle, néopentyle, phényle, benzyle, les alcoyles d'alcools gras de $C_{10}$ à $C_{22}$ et autres groupes similaires, et le polyoxyéthylène glycol méthoxylé comportant de 1 à 100 unités éthylèneoxy.

Lorsque R et $R^1$ sont reliés ensemble de façon à réaliser une liaison carbone-carbone saturée ou non, ces atomes de carbone peuvent être, ou non, substitués par des restes aliphatiques ou aromatiques. On donne ci-dessous quelques exemples non limitatifs de tels groupes ester-lactone

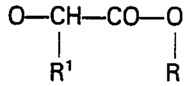

substitués ou non, correspondant à la définition susmentionnée: groupe diméthylène $-CH_2-CH_2-$; groupe diméthyléthylène $-CH(CH_3)-CH(CH_3)-$; groupe vinylène $-CH=CH-$; groupe triméthylène, $-(CH_2)_3-$, groupe méthyléthylène $-CH=CH-CH_2-$, groupe 1,2-phénylène

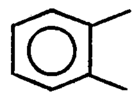 ;

cyclohexénylène

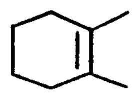 ;

groupe cyclopenténylène

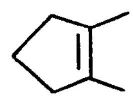 ;

cyclopentadiénylène

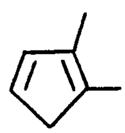 ,

groupe de formule

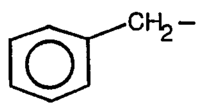

Le polymère de l'invention peut également se présenter sous la forme de coplymère avec d'autres polyaminoacides. Dans ce cas, on aura un copolymère de formule:

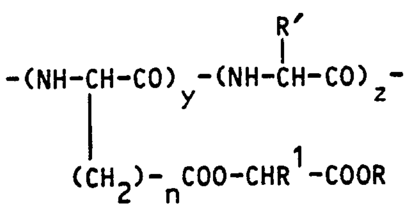 (II)

où R′ est un reste d'acide aminé non carboxylé ou carboxylé; dans ce dernier cas, les groupes COOH peuvent être libres, partiellement estérifiés ou totalement estérifiés, les groupes R′ des unités $-(NH-CHR'-CO)$ pouvant être identiques ou différents dans la chaîne du copolymère, avec $y + z = x$, la valeur de x étant toujours choisie pour que la copolymère ait une masse moléculaire moyenne d'au moins 5000 D. Bien entendu, si R′ est identique au groupe $-(CH_2)_n-COO-CR^1R^2-COOR$, avec des n cependant différents (l'un d'entre eux valant 1 et l'autre 2), on aura un copolymère estérifié d'acide glutamique et aspartique. Cependant, en règle générale, on préfère pour R′ avoir des groupements différents, tels que, par exemple méthyle (alanine), isopropyle (valine), isobutyle (leucine et isoleucine), benzyle (phenylalanine); R′ peut également désigner un reste d'acide glutamique ou aspartique non estérifié, ou estérifié partiellement par un alcool quelconque, par exemple MeOH ou EtOH, c'est-à-dire, par exemple, $-(CH_2)-_n-COOH$ ou $-(CH_2)_n-COOMe$. Il est à remarquer que si R′ désigne un reste d'acide glutamique ou aspartique libre, on peut représenter le polymère par la formule I, mais en admenttant que le degré de substitution (estérification) est inférieur à 100%; bien entendu, ce cas est également représentable par la formule II avec $R' = (CH_2)_n-COOH$ et $y/z+y$ étant égal au degré de substitution.

On pourra également avoir, indifféremment, des acides aminés de la série L ou D. Les acides aminés de la série L (ou naturels) sont les plus intéressant car les polypeptides les contenant sont dégradables par les enzymes (protéases) du coprps humain, alors que les polypeptides constitués d'unités D ne le sont pas. On peut mettre à profit cette différence grâce à des copolymères comprenant des aminoacides D et L, ceci afin de disposer de polymères dont la vitesse de dégradation est modifiée.

Revenant à des considérations plus générales, il faut noter que la proportion molaire, dans le copolymère II, de l'autre polyaminoacide libre ou partiellement estérifié permet également dans une notable mesure de régler la vitesse de biodégradation du copolymère en fonction des agents présents dans l'organisme au site de destination du mélange de copolymère et du médicament à adminstrer, (c'est-à-dire dans l'organe où le médicament doit agir). Ainsi, par exemple, si le coplymère est un copolymère de polyglutamate I et de leucine, on choisira la proportion molaire relative des deux constituants en fonction de la vitesse relative de dégradation, au lieu considéré, du polyglutamate et de la polyleucine. De préférence, le rapport z/y peut varier de 1 à 30. Bien entendu, dans le cas où le groupe R' ne désigne pas un groupe de nature unique dans la chaîne du copolymère, c'est-à-dire, par exemple, lorsque l'un des R' désigne un reste d'acide aminé libre et qu'un autre R' désigne un reste d'amino-acide estérifié, on pourra, pour plus de commodité désigner les variantes de R' par les signes R'', R''', etc.. La formule générale d'un tel copolymère peut alors être schématisée comme suit:

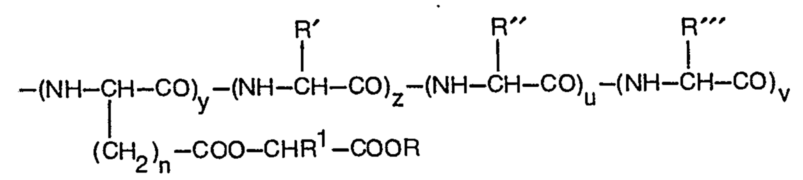

où la somme des y, z, u, v, . . . , etc est égale à x; u, v, etc peuvent être bien entendu nuls si le reste désigné par R' est de nature unique. Un cas typique où le copolymère présente des R' et R'' distincts est celui où ces groupes désignent des restes d'acide glutamique et/ou aspartique estérifiés et non estérifiés, la formule schématique d'un tel polymère (dans la cas d'espèce, partiellement méthylé) se présentant comme suit:

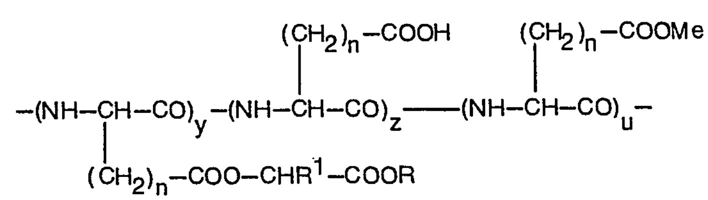

La nature du group R peut également influencer la vitesse de dégradation du polymère I. Ainsi, par exemple, si R est un groupe volumineux ou encombré (par exemple tert-butyle), la dégradation sera plus lente qu'avec un groupe méthyle ou éthyle.

Il est bien entendu que, du point de vue isomérie optique, les polymères de l'invention peuvent comprendre des éléments de configuration L ou d ou des mélanges racémiques ou, encore, des polymères où une des configurations domine. Les propriétés biochimiques de ces divers assemblages ne sont, bien évidemment, pas identiques, les polymères où dominent les formes naturelles (L) étant plus accessibles à la dégradation enzymatique. On peut donc en contrôler la dégradabilité en dosant, dans le copolymère les proportions relatives de l'une et l'autre forme.

Les polymères I et copolymère II sont insolubles dans l'eau et généralement solubles dans un ou plusieurs des solvants usuels tels que l'acétone, la méthyléthyl cétone (MEK), le tetrahydrofurane (THF), le dioxane, l'acétate d'éthyle, le monoglyme, le diglyme, ce qui permet leur transformation aisée en billes, bâtonnets, fibres, filaments, microcapsules, films, etc.. Les polymères I et II suivant leur structure peuvent être insolubles ou solubles dans les solvants chlorés, par exemple le chloroforme. Dans certains cas d'insolubilité dans le chloroforme, on peut y remédier si on ajoute à un tel solvant un peu d'acétone. Cette faculté de dissolution dans de nombreux solvants miscibles ou non miscibles à l'eau les rend également compatibles sans autre avec de nombreux médicaments liquides ou solubles dans les mêmes solvants. Ainsi, par exemple, on peut, pour encapsuler un produit hydrosoluble dans des microbilles de polymère, utiliser la technique connue consistant à disperser une solution aqueuse du médicament dans une solution de polymère, celle-ci comprenant un solvant non miscible à l'eau, puis à évaporer ce solvant de manière que se forment les capsules solides de polymère.

Par ailleurs, suivant sa structure, et plus particulièrement lorsque R est un groupe ramifié, par exemple t.Bu, le polymère I est très souvent parfaitement compatible avec les polyalcoylène glycols (polyéthylène glycol et polypropylène glycol), ce qui permet d'utiliser ces polyéthers glycol comme plastifiants du polymère I et de fournir ainsi un mélange homogène de bas point de fusion. On notera que, lors de l'utilisation du PEG comme solvant du polymère de l'invention, celui-ci contiendra une certaine humidité, de l'ordre de 5 à 50% en poids, laquelle permet une meilleure homogénéisation du mélange. On peut facilement incorporer toute une gamme de médicaments thermolabiles à un tel mélange (fusion à des températures de l'ordre de 40 à 60°C) et un obtenir des granulés ou des microcapsules. De plus, la présence de polyalcoylène glycols très hydrophiles permet d'augmenter la susceptibilité du polymère et du copolymère aux liquides aqueux biologiques et faciliter leur dégradation enzymatique in situ. Il est à remarquer que les polypeptides de synthèse connus ne possèdent pas ces propriétés favorables de solubilité et de compatibilité avec les PEG. Ainsi, par exemple, pour former des films d'acide

polyglutamique présentant une résistance mécanique appréciable et une certaine insolubilité dans l'eau, on doit utiliser des solutions dans des solvants relativement malaisés à manipuler et peu appréciés en pharmacie tels que diméthyl formamide (DMF) et acides dichloroacétique (DCA) et trifluoroacétique (TFA). Les films d'acide polyglutamique obtenus à partir de solutions aqueuses, (à pH 7,4, c'est-à-dire où l'acide est au moins en partie sous forme salifiée) n'ont aucune résistance mécanique et sont rapidement dissous dans l'eau, ce qui rend le polymère entièrement impropre en tant que support de médicament retard au sens de la présente invention. Il en est d'ailleurs de même es mélanges acide polyglutamique-polyethylène glycol que sont instantanément solubles dans l'eau. Lorsque le groupe R représente un reste d'alcool gras, par exemple un reste alcoyle de $C_{12}$ ou plus, le polymère est soluble dans les solvants chlorés et les esters, par exemple l'acétate d'éthyle; il est également oléosoluble même lorsque le degré de substitution est relativement peu élevé, 50% par exemple (voir formule II où R = $C_{12}$ alcoyle et R' = $(CH_2)_n$—COOH avec z = y). On peut ainsi former des solutions visqueuses ou pâteuses dans des huiles telles que l'huile de sésame ou le myristate d'isopropyle, ces solutions formant des émulsions stables avec l'eau. De telles émulsions premettent l'injection intra-musculaire de médicaments lipophiles (dissous dans la solution de polymère) dont le temps de relargage est contrôlable en fonction de la quantité dudit polymère en solution.

La biodégradation du polymère I peut être schématisée comme suit:

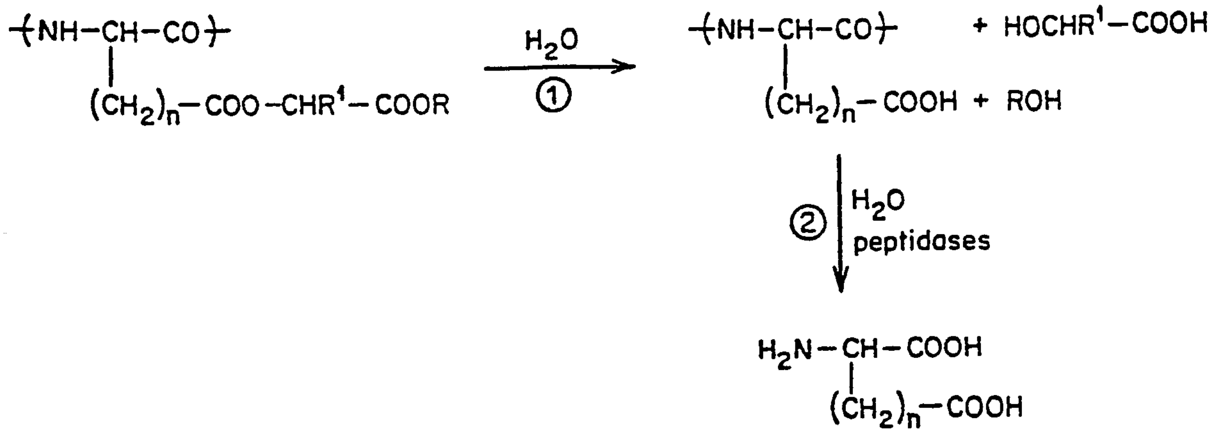


La réaction (2) est consécutive à la réaction (1) et, de ce fait, la biodégradation du polymère sera d'autant plus rapide que la vitesse d'hydrolyse de l'ester lactone ou hydroxyacétique est plus grande. Il est à noter que lorsque $R^1$ est un méthyle, le composé résultant de la réaction (1) est l'acide lactique, un produit biologiquement compatible. Plus le degré d'estérification est élévé, c'est-à-dire plus y est grand par rapport à z dans la formule II où R' = $(CH_2)_n$—COOH, plus la dégradation du polymère est lente comme on peut le déduire du schéma réactionnel précédent. Les produits résultant de la réaction (1) lorsque R et $R^1$ sont reliés l'un à l'autre sont également très avantageux en raison de leur toxicité négligeable. Ainsi, si l'association R—$R^1$ correspond aux groupes éthylène ou 1,2-phénylène, les produits de dégradation seront, respectivement, les acides vinyl- et phenyl-hydroxyacétiques lentement éliminés par l'organisme sans réactions secondaires.

On peut préparer le polymère I par estérification directe d'un sel de polyaminoacide correspondant avec un ester acétique α-halogéné (X—$CHR^1$—COOR (III)) ou x peut être le chlore, le brome ou l'iode. Le sel de polyaminoacide est, de préférence, un sel d'amine tertiaire (par exemple de tributylamine ou de triéthylamine). Une telle technique est connue en soi de par E. FALCH et al., J. Med. Chem. (1981) *24*, 285—289.

Le polyaminoacide ou co-polyaminoacide dont l'estérification fournit le polymère I ou le copolymère II s'obtient facilement par les moyens habituels comprenant l'estérification par un alcool inférieur du carboxyle latéral d'un acide de formule

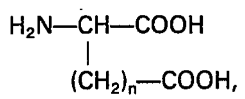

la transformation de l'ester en N-carboxyanhydride correspondant (NCA) par le phosgène en milieu dioxane ou THF, la polymérisation du NCA en polyaminoacide estérifié et l'hydrolyse du groupe ester protecteur en milieu alcalin ou par l'acide trifluoroacétique. De telles méthodes sont connues en soi (voir par exemple Encyclopedia of Polymer Science and Technology; N-carboxyanhydrides, vol II, page 837). Lorsqu'on désire parvenir à un copolymère ou R' désigne un carboxyle latéral partiellement estérifié (R' = —$(CH_2)_n$—COOH et R'' = —$(CH_2)_n$—COOAlk) on prendra soin que l'hydrolyse du groupe ester protecteur ne soit que partielle. Ainsi, par exemple, le produit de départ qu'on estérifiera avec le composé $XCHR^1$—COOR sera un copolymère d'acide $H_2N$—CH[$(CH_2)_n$—COOH]—COOH et d'ester $NH_2$—CH[$(CH_2)_n$—COO Alk]—COOH.

Les polymères et copolymères de la présente invention sont biodégradables et biocompatibles lors de

leur utilisation pour le relargage lent et contrôlé de médicaments par exemple à partir de films minces préparés par coulée d'une solution de polymère et de médicament sur un support suivie d'un séchage par évaporation des solvants de la solution. De telles techniques sont décrites dans "Controlled Release of Macromolecules from Polymers par R. LANGER et al., Biomedical Polymers, Ed, GOLDBERG et NAKAJIMA, Academic Press, 1980). Après séchage du film, le médicament peut se trouver à l'état dissous ou sous forme de suspension de particules.

En raison de leur solubilité dans le PEG, certains des polymères de l'invention peuvent être plastifiés par addition d'une faible quantité de ce polyol ce qui leur confère des propriétés thermoplastiques autorisant leur moulage à chaud suivant des formes diversifiées, par exemple fils, capsules, implants, très économiques à réaliser.

Par ailleurs, de tels mélanges avec le PEG permettent de régler la vitesse de biodissolution dans l'organisme du polymère en fonction du taux de PEG et de son poids moléculaire et, en conséquence, celle de relargage des médicaments incorporés. De plus, le pouvoir dissolvant de tels mélanges polymères-PEG vis-à-vis des médicaments est considérable et permet d'incorporer ceux-ci à des concentrations exceptionnellement élévées.

Le polymère I et le copolymère II sont utilisables comme réservoir de médicaments de diverses manières. Ainsi, par exemple, on peut employer les présents polymers I et copolymères II pour fabriquer des microcapsules contenant un médicament. De telles microcapsules comprennent une membrane polymérique et contiennent une solution aqueuse ou huileuse dans laquelle le médicament est en suspension ou en solution. On peut également fabriquer des microsphères, c'est-à-dire des particules solides ou billes content le médicament à l'étet dispersé ou à l'état de solution solide dans la matrice de polymère. On peut également fabriquer des produits microporeux dénommés microéponges. En général, on pourra mettre en oeuvre, au moyen des présents polymères, toutes les techniques de fabrication de médicaments retard, c'est-à-dire ayant la propriété de relâcher (relarguer) le médicament de manière prolongée au fur et à mesure de la dégradation du support. On trouvera une description de ces techniques dans les ouvrages suivants: "Biodegradables and Delivery Systems for Contraception", Mafez E.S.E., MTP Press Limited (1980); "Controlled Release Technologies = Methods, Theory and Applications" Vol. 1 et 11, A. F. Kydonieus, CRC Press (1980) et "Microencapsulation — New Techniques and Applications" par Tamotsu KONDO, Techno Inc. (1979) Japan. Le solubilité des présents polymères dans de nombreux solvants, miscibles à l'eau ou non, est un avantage pour leur application selon les techniques décrites dans ces références. Il est également possible de préparer des fils constitués de ces polymères en extrudant une solution de ceux-ci dans une fillière et en précipitant le fil soit par évaporation, soit par un bain de non solvant, selon les techniques habituelles de filage. Des filaments préparés ainsi peuvent être tricotés, noués ou tissés pour former des sutures, des ligatures ou des structures tubulaires pouvant servir d'artères artificielles, de veines, de conduits ou d'organes internes à fonctionnement temporaire. Les polymères de l'invention peuvent également servir, soit directement, soit en mélange avec un plastifiant, à la fabrication de films ou de prothèses chirurgicales servant, par exemple, à la consolidation d'os fracturés, comme des agrafes, des aiguilles, des vis, des plaques de renforcement, des tampons, etc., ces matériaux pouvant être réalisés par coulage ou moulage de solution, thermoformage ou par usinage de blocs de polymère solides. De telles prothèses étant résorbables, elles sont progressivement éliminées dans l'organisme et il n'est alors plus nécessaire de prévoir, comme on le fait actuellement, une nouvelle opération pour enlever le matériau de renforcement et de consolidation.

Les polymères et copolymères de l'invention sont également utilisables pour la préparation de pansements chirurgicaux biodégradables. De tels pansements sont constitués par une ou plusieurs couches successives obtenues à partir de solutions de ces polymères dans un solvant hydrocompatible, par exemple PEG, DMF, acétone, méthyl-éthylcétone, butanol, éthanol, dioxanne, tetrahydrofuranne, déposées sur un support et solidifiées par extraction à l'eau du solvant en question. Une telle extraction peut se faire par mise en contact avec de l'eau, par exemple par lavage ou immersion. De telles solutions peuvent en outre contenir un agent porogène, notamment un sel hydrosoluble ou un solvant volatil. Lors du traitement par l'eau du film de polymère, les grains de ce sel confèrent à celui-ci se dissolvant une structure poreuse. De même, par évaporation du solvant volatil, après lyophylisation de la couche de revêtement.

On peut constituer de tels pansements par coulées des solutions sur un support (ces solutions contenant, ou non, un ou plusieurs médicaments, par exemple un désinfectant) dans des conditions stériles, en traitant le tout à l'eau puis en détachant ensuite le film insolubilisé du support et le séchant éventuellement avant emploi (ou en l'emballant de manière stérile si son utilisation immédiate n'est pas prévue).

On peut également, et c'est là un mode d'utilisation avantageux, notamment dans le cas des solutions du présent polymère où R est une reste ramifié ou d'alcool gras dans le PEG, directement couler une ou plusieurs solutions (sous forme de liquides visqueux ou de pommades) sur la plaie à panser et ensuite, comme ci-dessus, procéder à la solidification (insolubilisation) du pansement par traitement à l'eau de la région pansée (aspersion, immersion du membre pansé dans un bain d'eau ou autre). Une telle technique permet d'assurer une étanchéité excellente entre la plaie et l'air extérieur et de minimiser les risques d'infection. Par ailleurs, vu la biodégradabilité du film de pansement, il n'y a pas lieu de prévoir son arrachage, sa résorbtion s'effectuant de concert avec la cicatrisation.

Bien entendu, la composition exacte du polymère ou copolymère utilisé est à régler, en fonction des vitesses de dégradation et des caractéristiques d'absorption in vivo, suivant la nature de la prothèse envisagée.

Les exemples qui suivent illustrent l'invention.

Exemple 1

Polyglutamate de tert-butyloxycarbonylméthyle

On a préparé de l'acide polyglutamique (PGA) à partir du N-carboxyanhydride de γ-glutamate de méthyle dissous dans le chlorure de méthylène; on a utilisé la triéthylamine comme initiateur de polymérisation (A/I=100). On a ensuite précipité le polymère par adjonction de méthanol, puis on l'a séché sous vide. On a redissous le solide dans de l'acide trifluoroacétique (TFA) de manière à réaliser une solution à 5% en poids et on a ajouté goutte à goutte en agitant vigoureusement une volume d'eau distillée suffisant pour que la soluion finale contienne des volumes égaux d'eau et de TFA. On a maintenu encore 24 h. en agitation à température ambiante (solution visqueuse) après quoi on a versé le tout sur de l'eau distillée en grand excès, ci qui conduit à la précipitation de l'acide plyglutamique. On a filtré cet acide et on l'a séché. On a contrôlé la pureté de l'acide ainsi obtenu par analyse RMN dans la TFA (absence de la bande ester méthylique —O—$CH_3$ à 4,5 ppm).

On a dissous 2,5 g d'acide polyglutamique (0,019 moles, viscosité relative mesurée 2,6) dans 100 ml de diméthylformamide (DMF), puis on a ajouté successivement 3,78 g (0,019 moles) de tributylamine et 3,7 g d'α-bromoacétate de tert-butyle. Après 24 heures sous agitation, on a dilué le tout à l'eau acidulée (HCl 0,05 M) de manière à faire précipiter le polymère désiré. On a redissous celui-ci dans 200 ml d'acétone et on l'a fait reprécipiter par dilution dans 3 l d'HCl 0,05 M. On a ensuite répété ce traitement de purification par redissolution dans 200 ml d'acétone et précipitation à l'éther. Après séchage, on a obtenu 2 g (43%) du polymère de formule

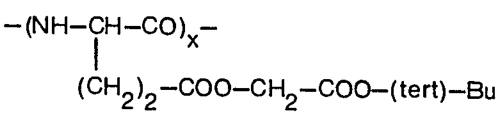

L'analyse par RMN dans l'acide trifluoracétique (TFA) a fourni les données suivantes: δ (t.butyle) 1,35 ppm, 9H; δ 2—3 ppm, —$CH_2$— α et β; δ 5 ppm 3H, α—CH et O—$CH_2$—CO; δ = 8 ppm 1H, —NH—. Par intégration, on a constaté que le polymère était estérifié à 75%. En conséquence, il serait possible de représenter la produit ainsi obtenu par la formule de type II suivante

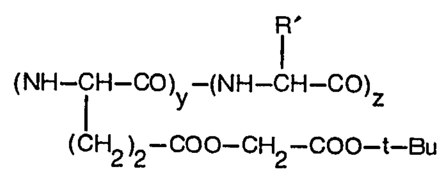

où R' est un reste $(CH_2)_2$—COOH et y = 3z. Ce polymère est soluble dans les solvants suivants: acétone, THF, DMF, polyethylène glycol (PEG—400). Il forme des gels insolubles dans les solvants chlorés et le benzène. A noter que pour une bonne dissolution dans le PEG, le présent polymère ne doit pas être séché complètement mais conserver, après purification, une certaine teneur en humidité résiduelle (par exemple de 5 à 50% en poids). Un polymère complètement sec ne se redissout pas directement dans le PEG; il faut préalablement le dissoudre dans le TFA et le reprécipiter par exemple à l'eau. On notera que le degré d'estérification du groupe —COOH (75% dans le cas présent) peut être modifié en fonction de la quantité d'α-bromoacétate utilisé; ainsi, avec 0,5 équivalent de ce réactif, le degré de substitution est de 30% environ, alors qu'avec 2 équivalents, on aittient 85% de substitution.

En remplaçant, dans l'exemple ci-dessus, l'acide polyglutamique par de l'acide polyaspartique, on obtient le polyaspartate correspondant dont les propriétés sont similaires.

Exemple 2

Polyglutamate de 2-hydroxybutyrolactone

On a procédé comme dans l'Exemple 1 à partir de 2 g d'acide polyglutamique (PGA), 35 ml de DMF, 5,74 g de tributylamine (2 équiv), 5 g d'$H_2O$ et 5,12 g d'α-bromobutyrolactone. Après 4 jours sous agitation, on a précipité à l'eau redissous au DMF et reprécipité à l'éther. Après séchage, on a effectué une analyse par IR dans le K Br avec les résultats suivants: $\nu_{(co)}$, 1760 $cm^{-1}$ (carbonyle lactonique); $\nu_{(co)}$, 1720 $cm^{-1}$ (carbonyle carboxylique). Le polymère se dissout dans la soude caustique 0,1N d'où on peut le titre à rebours par le HCl 0,1N (virage à pH 2—3). La titration correspond à la reformation de l'ester polyglutamique hydroxycarboxylique IIC ci-dessous à partir du sel de sodium correpondant, lui-même

résultant de l'ouverture du cycle lactonique par dissolution dans la soude du polymère recherché IIB. L'acide IIC est un acide assez fort (pK ~ 1) reformant lentement la lactone de départ dans l'eau à pH 2—3 (voir schéma ci-dessous).

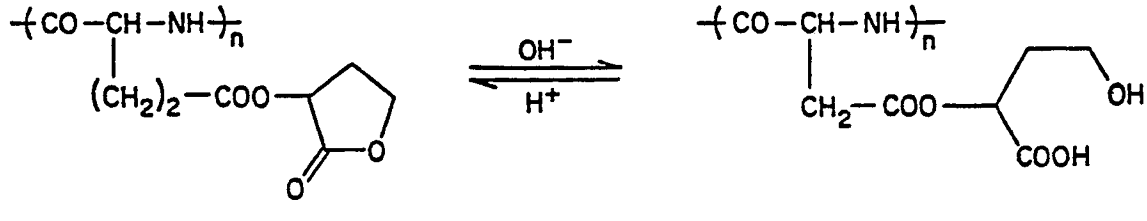


II B    II C

Le polyglutamate de 2-hydroxybutyrolactone est soluble dans les solvants suivants: DMF, TFA, acide dichloracétique (DCA); il est insoluble dans les solvants suivants: acétone, chloroforme, méthanol, eau.

Exemple 3

Polyglutamate d'α-hydroxyvalerolactone

On a procédé comme dans les Exemples précédents à partir de 1 g de PGA (7,8 mMoles) 40 ml de DMF, 2,87 g (15,5 mMoles) et 2,77 g (15,5 mMoles) d'α-bromo-γ-valerolactone. Après 48 heures d'agitation à température ambiante, on a précipité le polymère par dilution à l'eau, puis on l'a redissous dans l'acétone et reprécipité à l'éther. Par analyse en RMN, on a constaté, par intégration des pics correspondant au proton amino (δ = 8 ppm) et aux protons CH—$CH_3$ (δ = 0,35 ppm) que le degré d'estérification était d'environ 90%. Le spectre IR confirme la présence du pic lactonique ($\nu_{(CO)}$ = 1760 $cm^{-1}$).

Le produit de formule

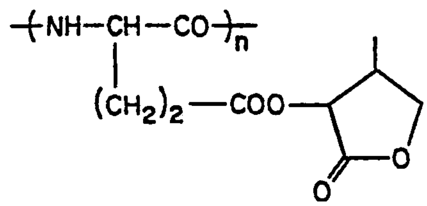

est soluble dans les solvants suivants: TFA, DMF, acétone, $CHCl_3$, $CH_2Cl_2$, etc.. et insoluble dans les solvants suivants: EtOH, MeOH, méthyl-éthyl cétone (MEK), acétate de 2-méthoxyéthyle (AME), acétate d'éthyle.

Exemple 4

Polyglutamate de méthoxycarbonylméthyle

On a procédé comme dans les Exemples précédents à partir d'l equivalent de chacun des réactifs suivants, le tout dans une quantité de solvant (DMF) correspondant à 50 parties de PGA: PGA; $Bu_3N$; $BrCH_2$—COOMe. On a ainsi obtenu, avec un rendement de 60%, le produit de formule

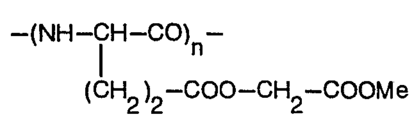

dont les propriétés sont les suivantes: solubilité dans les solvants suivants: acétone, THF, dioxane, DMF. La solubilité est imparfaite dans le PEG 400 pur; on obtient dans ce cas des quasi-solutions opalescentes ayant tendance à former des gels. En présence d'alcools inférieurs, le polymère subit un gonflement mais ne se dissout pas.

Exemples 5 à 8

On a répété la procédure des Exemples précédents à partir de PGA (1 équiv.) et des composés halogénés (1 équiv.) figurant au Tableau ci-dessous, le tout par agitation dans le DMF 48—100 heures à température ambiante. La purification des produits a été effectuée par redissolution et reprécipitation comme décrit auparavant.

| Exemple | Ester halogéné | Produit obtenu = polyglutamate de |
|---|---|---|
| 5 | BrCH—COOEt | -éthoxy-carbonylméthyl |
| 6 | $BrCH_2$—COOBz | -benzyloxycarbonylméthyle |
| 7 | $BrCH_2$—COOPh | -phénoxycarbonylméthyle |
| 8 | $BrCH(CH_3)$—COOMe | -méthoxycarbonyl-1-éthyle |

Les propriétés de solubilité de ces produits sont les suivantes:

| Exemple | Solvants | non solvants |
|---|---|---|
| 5 | acétone, dioxane THF, DMF | $CHCl_3$, $CH_2Cl_2$, Bz |
| 6 | acétone, AcOEt, $CHCl_3$, dioxane | Bz |
| 7 | acétone, dioxane THF, DMF | solvants chlorés |
| 8 | comme à l'Exemple 5 | comme à l'Exemple 5 |

Exemple 9

Copolymère de polyglutamate de tert-butoxycarbonylméthyle et de leucine (50:50)

On a préparé ce copolymère d'une manière similaire à celle décrite à l'Exemple 1 en replaçant, en tant que produit de départ, l'acide polyglutamique par un copolymère statistique (50:50 en poids) de L'leucine et d'acide L-glutamique, préparé lui-même par copolymérisation des N-carboxy-anhydrides correspondants. La structure du copolymère obtenu a été établie par analyse suivant les moyens habituels (spectres IR et de RMN). On a préparé des films minces (0,1 mm) de ce polymère en étalant sur une plaque de verre une couche de sa solution acétonique (à 10% en poids) et en évaporant le solvant. Ces films ont été ensuite soumis à une dégradation dans une solution de protéase, en compagnie de films similaires préparés à partir du polymère de l'Exemple 1. On a constaté que le dégradation des films de copolymère était plus lente que celle des films de polymère seul.

Exemple 10

Copolymère de polyaspartate de methoxycarbonylméthyle et de leucine

On a procédé comme ci-dessus, mais en utilisant, comme produit de départ, un copolymère d'acide aspartique et de leucine obtenu de manière similaire. On a également préparé, à titre comparatif, un polyaspartate de tert-butoxycarbonylméthyle suivant la méthode de l'Exemple 1. Les propriétés de ces polymères et copolymères étaient très voisines de celles des polymères glutamiques correspondants.

Exemple 11

A une solution de 2 g du polymère suivant l'Exemple 1 dans 300 ml d'acétone, on a ajouté 30 ml de PEG—400 (Ph. Helv. IV) et on a soumis cette solution à une évaporation sous vide jusqu'à formation d'une solution très visqueuse à consistance de pommade (600—700 P). On a étalé une couche d'environ 0,2—0,5 mm de cette pâte sur une plaie pratiquée dans la peau d'un rat d'expérience puis on a aspergé deux min. d'eau stérile, la zone ainsi revêtue. Il se forme alors un film souple et résistant épousant parfaitement la forme de la plaie et ne présentant aucune fissure visible après 24 heures en dépit des mouvements de l'animal traité. Un tel film, obtenu sur un support inerte (plaque de verre) par des moyens identiques, présente une preméabilité à la vapeur d'eau à température ambiante de 4 mg/h.$cm^2$.

Après deux semaines, on a constaté que la plaie s'était parfaitement cicatrisée, le film protecteur s'étant, de son côté, résorbé.

Exemple 12

Fabrication d'un implant biodégradable contenant un médicament relargable

On a dissous 1 g du polymère suivant l'Exemple 1 et 1 g de Diazepam (Roche) dans un mélange de 200 ml de PEG 400 et 50 ml d'acétate de 2-méthoxyéthyle. On soumet cette solution à une évaporation lente à pression ordinaire, puis sous pression réduite $1{,}3322.10^{-2}$ Pa; 40°C) jusqu'à obtention d'un produit pâteux qu'on extrude ensuite en fils de 0,9 mm dans une filière ($2.10^6$ PA; 60°C).

On a étudié cette pâte par calorimétrie différentielle à balayage (différentiel Scanning Calorimetry,

DSC) et on a constaté, par absence des pics correspondant à la fusion (130°C) du Diazepam, qu'il s'agissait là d'une solution vraie. Il est à noter dans ce contexte qu'en omettant le PEG dans la solution de départ, on obtient après évaporation un produit d'aspect général similaire mais non-extrudable aux conditions précitées et contenant le Diazepam à l'état partiellement cristallisé.

Après avoir refroidi le fil à −5°C pour en augmenter la rigidité, on en a introduit un fragment de 5 cm (32 mg, 14,5 mg de médicament) dans l'aiguille (1 mm de ϕ) d'une seringue à injections. On a ensuite injecté (implanté) ce tronçon sous le peau d'un rat de laboratoire (WISTAR mâle, 350 g). Pour ce faire, on a introduit l'aiguille sous la peau de l'animal sur une longueur légèrement supérieure à celle du tronçon à implanter, puis on a retiré celle-ci tout en pressant sur le mandrin de la seringue de manière que le fragment de fil reste en place au fur et à measure du retrait de l'aiguille.

On a ensuite prélevé périodiquement des échantillons sanguins de l'animal et on les a analysés par GPC (Chromatographie en phase gazeuse) suivant I. A. ZINGALES (J. of Chromatography *75* (1973), 57—59) pour déterminer la teneur en Diazepam. On a utilisé un chromatographe Hewlett-Packard 5710—A, un colonne SUPELCO SP—2250 et un détecteur par capture d'électrons. Ces analyses ont montré que la concentration plasmatique de Diazepam tend vers un maximum (après 48 h) de 1800 ng/ml, puis redescend lentement jusqu'à 100—200 ng/ml après 10 jours. A ce propos, on note que, pour être efficace phamacodynamiquement, une telle concentration doit être comprise entre 180 ng et 3 µg/ml suivant l'effet recherché.

La présente expérience ayant été faite sur plusieurs animaux, on a sacrifié certains de ceux-ci à différentes périodes après l'implantation et on a constaté qu'après 12 jours la matière de l'implant était fortement dégradée et en voie de résorption par les tissus adjacents.

On notera encore à titre comparatif qu'après injection sous cutanée de Diazepam en solution hydro-alcoolique suivant la pratique habituelle, l'effet du médicament ne dure que 5 heures environ.

## Exemple 13

On a utilisé du polyglutamate de tert-butoxycarbonylméthyle préparé suivant l'Exemple 1 et contenant, après précipitation, environ 20—30% d'humidité résiduelle. On a préparé une solution à 5% en poids de ce polymère dans du PEG—400 et on a préparé des films d'environ 0,1 à 0,2 mm d'épaisseur en coulant la solution sur un support (plaque de verre) et en lavant ensuite celle-ci à l'eau (par immersion dans un lent courant) pendant des périodes variables allant de 4 heures à 24 heures.

Les films ainsi obtenus contenaient des quantités variables de PEG retenu dans la matière du polymère (d'environ 0,1 à 5% suivant le temps de lavage).

On a trempé ces films dans une solution tampon 0,05 M, pH 8,5 température ordinaire et on a constaté que la dégradation de ces films (hydrolyse) variait en fonction du contenu en PEG résiduel, le temps minimum observé (cas du taux le plus élevé en PEG) étant de 3 heures et le temps maximum de 48 heures.

Les essais ci-dessus ont été répété en utilisant, comme milieu de dégradation, une solution de leucine aminopeptidase à 8,5 U/ml dans un tampon (pH 8,5) 0,1 M borate et 0,005 M $MgCl_2$. Dans ces conditions, les vitesses d'hydrolyse ont été pratiquement doublées.

## Exemple 14

Polyglutamate de dodecyloxycarboxyl méthyle.

On dissout 7,00 g (0,0542 moles) d'acide polyglutamique dans 150 ml de DMF. On ajoute 2 équivalents de tributylamine (TBA) (20 g, 0,108 moles) et 2 équivalents de bromoacétate de dodécanyle (33 g, 0,108 moles) préparé par estérification de l'acide bromoacétique par le dodecanol en présence de dicyclohexyl-carbodiimide (DCC). Au bout de 20 minutes la solution se trouble. On redissout le précipité en rajoutant 50 ml de chloroforme puis 250 ml d'éther. Après 5 jours de réaction, le mélange réactionnel est évaporé au rotavapor jusqu'à 150 ml et on précipite le tout dans un litre de MeOH. Le polymère est redissous dans l'éther et précipité par l'hexane. Il est soluble dans l'éther éthylique, $CH_2Cl_2$, $CHCl_3$, le myristate d'isopropyle, les huiles de sésame et de tournesol, l'acétate d'éthyle. Il est insoluble dans le DMF, l'acétone, l'eau, l'éther de pétrole. L'analyse du polymère par RMN confirme la présence de groupes dodecyl. Le taux d'estérification est mesuré par chromatographie en phase gazeuse de l'hydrolysat: on porte 2 heures au reflux dans 7 ml de NaOH 5N, 105 mg de polymère. On acidifie ensuite à pH 2—3 avec HCl 35%; on extrait le dodecanol obtenu avec 4 × 15 ml d'éther et on injecte un aliquot du mélange dans un chromatographe en phase gazeuse; l'analyse montre un taux de substitution de 51%.

## Exemple 15

Polyglutamate de [poly(oxyéthylène)methyléther]oxycarbonylméthyle.

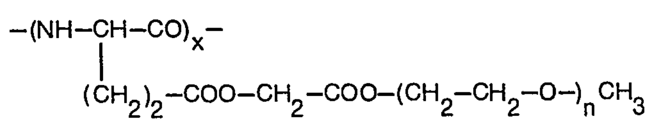

1. On prépare tout d'abord du bromoacétate de poly(oxyéthylène) monométhyl-éther de la manière suivante:

Dans 200 ml d'acétate d'éthyle, on dissout 24,6 g (0,177 moles) d'acide bromoacétique. On rajoute 31 g de polyéthylèneglycol 350 (PEG 350, Fluka), n ≅ 8, 1 g de 4-pyrrolidinopyridine. On refroidit le mélange à −4°C et on ajoute goutte à goutte 36,5 g de DCC (0,177 moles) dissous dans 100 ml d'acétate d'éthyl. Après 24 heures, on filtre, on évapore le filtrat jusqu'à obtention d'une huile qu'on extrait avec 5 × 200 ml d'éther de pétrole pour élimine l'excès d'anhydride bromoacétique et la 4-pyrrolidinopyridine. Le spectre RNM du produit obtenu correspond à la formule

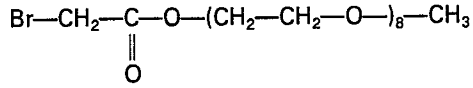

δ = 3,35 ppm ($OCH_3$) δ = 3,6 ppm ($CH_2-CH_2-O$) et δ = 3,90 ppm ($Br-CH_2-CO$).

2. On prépare le polymère en faisant réagir 2 g de PGA avec 15 g de bromoacétate de polyéthylèneglycol monométhyléther et 5,6 g de TBA dans 75 g de DMF. Après 5 hours de réaction, on précipite le polymère par $H_2O$, on le redissout dans du méthanol et on le précipite par l'éther. Le spectre RMN du polymère obtenu confirme la présence de résidus éthylène-oxide (δ = 3,6 ppm) le long de la chaîne polypeptidique. Le polymère est soluble dans le méthanol, l'acétone, le chloroforme et les polyéthylèneglycols. Il est insoluble dans l'eau, l'éther et l'éther de pétrole.

On notera, à propos du présent exemple, l'existence de composés analogues directement dérivés du PGA, les polyglutamates de polyéthylèneglycols monométhyléthers (voir Japan Kokai 59—149927). Les propriétés de biodégradabilité de ces composés ne sont cependant pas connues et, compte tenu de la structure de ces composés, elles devraient être inférieures à celles de composés de la présente invention.

**Revendications pour les Etats contractants: BE CH DE FR GB IT LI LU NL SE**

1. Polypeptide constitué de polyaspartate ou polyglutamate d'alcoyloxycarbonylméthyle ou d'aryloxycarbonylméthyle biodégradable et insoluble dans l'eau de formule

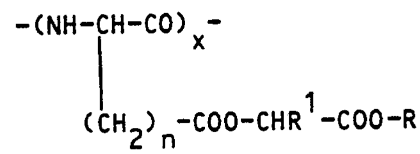

(I)

dans laquelle $R^1$ est un alcoyle inférieur ou l'hydrogène, R étant un reste aliphatique, notamment polyoxyalcoylénique hydroxylé ou alcoxylé (linéaire ou ramifié), ou aromatique, substitué ou non; ou bien R et $R^1$ sont liés l'un à l'autre en constituant un pont hydrocarboné substitué ou non à 2 ou 3 maillons; ou ses copolymères avec des acides aminés, de formula

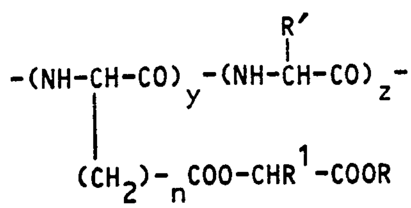

(II)

où les groupes R et $R^1$ ont le sens donné ci-dessus et où R′ est un reste d'amino-acide non carboxylé ou carboxylé dont le —COOH est libre ou partiellement ou totalement estérifié; n vaut 1 ou 2 et x qui est égal à y + z est choisi pour que la masse moléculaire du polypeptide ne soit pas inférieure à 5000 D.

2. Polypeptide suivant la revendication 1, caractérisé par le fait que R est choisi parmi les restes méthyle, éthyle, isopropyle, isobutyle, tert-butyle, néopentyle, phényle, benzyle, les alcoyles gras en $C_{10}$ à $C_{22}$ et le polyoxyéthylène glycol méthoxylé comportant de 1 à 100 unités éthylèneoxy.

3. Polypeptide suivant la revendication 1, caractérisé par le fait que, R et $R^1$ étant liés l'un à l'autre, le maillon formé par ceux-ci est choisi parmi les formules suivantes: $—CH_2—CH_2—$; $—CH=CH—$; $—CH(CH_3)—CH(CH_3)$; $—C(CH_3)=C(CH_3)—$; 1,2-phénylène; cyclohexénylène; cyclopenténylène, cyclopentadiénylène; triméthylène;

$-CH=CH-CH_2-$; 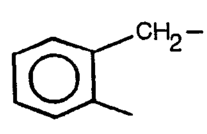 .

4. Polypeptide suivant la revendication 1, caractérisé par le fait que le composé (I) est choisi parmi les glutamate et aspartate d'alcoyloxycarbonyl- et aryloxycarbonylméthyle que le composé II est choisi parmi

les copolymères de glutamate ou aspartate d'alcoyloxycarbonyl- et d'aryloxycarbonylméthyle avec un ou plusieurs autres acides aminés choisis parmi l'alanine, la leucine, le valine et la phénylalanine.

5. Polypeptide suivant la revendication 1, caractérisé par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

6. Polypeptide suivant la revendication 4, caractérisé par le fait que les groupes alcoyle ou aryle R sont choisis parmi les groupes méthyle, éthyle, propyle, isopropyle, butyle, isobutyle, neopentyle, benzyle, phényle lauryle, cétyle, oléyle et stéaryle.

7. Utilisation du polypeptide I et ses copolymères II suivant la revendication 1 comme réservoir de médicament à effet retard dans l'organisme, ce dernier étant progressivement libéré à son lieu de destination consécutivement à la biodégradation du polymère porteur.

8. Utilisation suivant la revendication 7, caractérisé par le fait qu'on mélange ledit médicament et ledit polymère de façon homogène et qu'on façonne ce mélange sous une forme pharmaceutiquement acceptable.

9. Utilisation suivant la revendication 8, caractérisée par le fait qu'on mélange au polymère un polyalcoylène glycol de façon à le plastifier, qu'on y ajoute, un médicament puis qu'on moule par la chaleur le produit thermoplastique ainsi obtenu sous forme de granulés, bâtonnets, capsules ou autres particules aptes à être administrées par toutes voies habituelles.

10. Utilisation des polymères I et copolymères II, suivant suivant la revendication 1, pour fabriquer des implants et prothèses biodégradables utilisables en chirurgie.

11. Procédé de préparation d'un polypeptide constitué de polyaspartate ou polyglutamate d'alcoyloxycarbonylméthyle ou d'aryloxycarbonylméthyle biodégradable et insoluble dans l'eau de formule

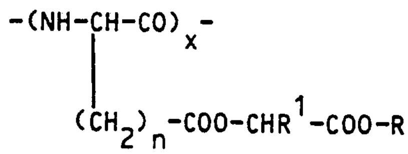

(I)

dans laquelle $R^1$ est un alcoyle inférieur ou l'hydrogène, R étant un reste aliphatique ou aromatique substitué ou non; ou bien R et $R^1$ sont liés l'un à l'autre de manière à consituer un pont à 2 ou 3 maillons d'atomes de carbone substitué on non; ou de ses copolymères avec d'autres acides aminés de formule

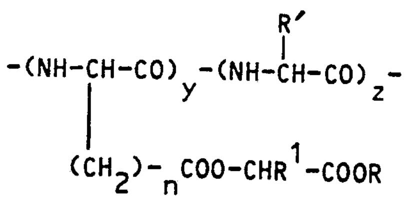

(II)

où les groupes R et $R^1$ ont le sens donné ci-dessus et où R' est un reste d'amino-acide libre ou partiellement ou totalement estérifié; n vaut 1 ou 2 et x qui est égal à y + z est choisi pour que la masse moléculaire du polypeptide ne soit pas inférieure à 5000 D, caractérisé par la fait qu'on fait réagir le polyaminoacide correspondant ou le co-polyaminoacide correspondant sous forme de son sel avec une amine tertiaire avec l'ester d'halogénométhyle.

$$X—CHR^1—COO—R \qquad (III)$$

où X = Cl, Br ou I et R et $R^1$ répondent aux définitions précitées, le composé I ou II se formant avec élimination simultanée du sel d'amine de l'acide hydrohalogéné correspondant.

12. Matière polymérique filmogène utilisable comme pansement, caractérisée par le fait qu'elle consiste en une solution dans un solvant organique hydrosoluble d'un polypeptide constitué de polyaspartate ou polyglutamate alcoyloxycarbonylméthyle ou d'aryloxycarbonylméthyle biodégradable et insoluble dans l'eau de formule

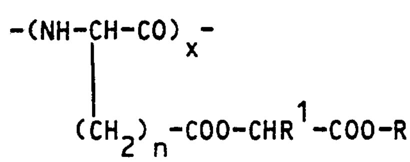

(I)

dans laquelle $R^1$ est un alcoyle inférieur ou l'hydrogène, R étant un reste aliphatique ou aromatique substitué ou non; ou bien R et $R^1$ sont liés l'un à l'autre de manière à constituer un pont à 2 ou 3 maillons d'atomes de carbone substitué ou non; ou de ses copolymères avec d'autres acides aminés de formule

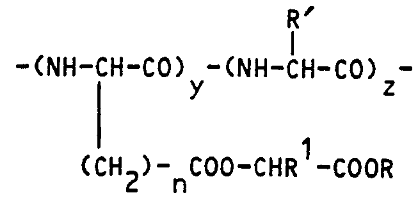

(II)

où les groupes R et $R^1$ ont les sens donné ci-dessus et où R' est un reste d'amino-acide libre ou partiellement ou totalement estérifié; n vaut 1 ou 2 et x qui est égal à y + z est choisi pour que la masse moléculaire du polypeptide ne soit pas inférieure à 5000 D.

13. Matière polymérique filmogène suivant la revendication 12, caractérisée par le fait que R est choisi parmi les restes méthyle, éthyle, isopropyle, isobutyle, néopentyle, tert-butyle, phényle, benzyle et les alcoyles gras en $C_{10}$ à $C_{22}$.

14. Matière polymérique filmogène suivant la revendication 12, caractérisée par le fait que, R et $R^1$ étant liés l'un à l'autre, le maillon formé par ceux-ci est choisi parmi les formules suivantes $—CH_2—CH_2—$; $—CH=CH—$; $—CH(CH_3)—CH(CH_3)$; $—C(CH_3=C(CH_3)—$; 1,2-phénylène; cyclohexénylène; cyclopenténylène; cyclopentadiénylène; $—(CH_2)_3—$; $—CH=CH—CH_2—$;

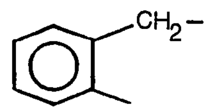

.

15. Matière polymérique filmogène suivant la revendication 12, caractérisée par le fait que la composé (I) est choisi parmi les glutamate et aspartate d'alcoyloxycarbonyl- et aryloxycarbonylméthyle et que le composé (II) est choisi parmi les copolymères de glutamate ou aspartate d'alcoyloxycarbonyl- et d'aryloxycarbonylméthyle avec un ou plusieurs autres acides aminés choisis parmi l'alanine, la leucine, la valine et la phénylalanine.

16. Matière polymérique filmogène suivant la revendication 12, caractérisée par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

17. Matière polymérique filmogène suivant la revendication 15, caractérisée par le fait que les groupes alcoyle sont choisis parmi les groupes méthyle, éthyle, propyle, isopropyle, butyle, isobutyle, tert-butyle, néopentyle, benzyle, phényle, lauryle, cétyle, stéaryle et oléyle.

18. Utilisation de la matière polymérique filmogène suivant la revendication 12, caractérisée par la fait que

on applique une couche de cette solution sur une surface à revêtir,

puis on traite cette surface ainsi revêtue par de l'eau, celle-ci ayant pour effet de dissoudre et d'éliminer le solvant hydrosoluble de manière à former le film de matière filmogène.

19. Utilisation de la matière polymérique filmogène suivant la revendication 12 comme réservoir de médicament à effet retard dans l'organisme, ce dernier étant progressivement libéré à son lieu de destination consécutivement à la biodégradation du film de polymère porteur.

20. Matière suivant la revendication 12, caractérisée par le fait que le solvant est un polyalcoylène glycol, par exemple le polyéthylène glycol (PEG).

21. Matière suivant la revendication 12, caractérisée par le fait que le solvant est au moins l'un des solvants suivants: PEG, diméthylformamide (DMF), acétone, méthyléthylcétone (MEK), butanol, éthanol dioxanne, tetrahydrofuranne (THF).

22. Matière suivant la revendication 12, caractérisée par le fait que ladite solution contient, en outre, un agent porogène.

23. Matière suivant la revendication 22, caractérisée par le fait que cet agent porogène est le sel moulu très fin, la dissolution des grains de celui-ci dans l'eau de traitement donnant lieu à la formation d'une structure de mousse poreuse.

24. Matière suivant la revendication 22, caractérisée par le fait que cet agent porogène est un solvant organique volatil et qu'on soumet la couche de revêtement à une lyophilisation de manière que, par évaporation du solvant volatil, il se forme dans la masse une structure poreuse.

**Revendications pour l'Etat contractant: AT**

1. Procédé de préparation d'un polypeptide constitué de polyaspartate ou polyglutamate d'alcoyloxycarbonylméthyle ou d'aryloxycarbonylméthyle biodégradable et insoluble dans l'eau de formule

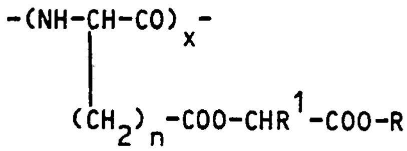

(I)

dans laquelle $R^1$ est un alcoyle inférieur ou l'hydrogène, R étant un reste aliphatique ou aromatique substitué ou non; ou bien R et $R^1$ sont liés l'un à l'autre de manière à constituer un pont à 2 ou 3 maillons d'atomes de carbone substitué ou non; ou de ses copolymères avec d'autres acides aminés de formule

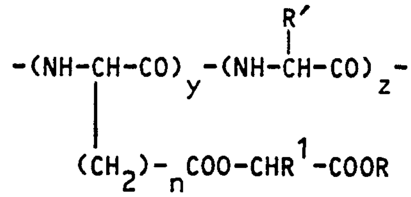

(II)

où les groupes R et $R^1$ ont le sens donné ci-dessus et où R' est un reste d'amino-acide libre ou partiellement ou totalement estérifié; n vaut 1 ou 2 et x qui est égal à y + z est choisi pour que la masse moléculaire du polypeptide ne soit pas inférieure à 5000 D, caractérisé par le fait qu'on fait réagir le polyaminoacide correspondant ou le co-polyaminoacide correspondant sous forme de son sel avec une amine tertiare avec l'ester d'halogénométhyle

$$X—CHR^1—COO—R \qquad (III)$$

où X = Cl, Br ou I et R et $R^1$ répondent aux définitions précitées, le composé I ou II se formant avec élimination simultanée du sel d'amine de l'acide hydrohalogéné correspondant.

2. Procédé suivant la revendication 1, caractérisé par le fait qu'on mélange un médicament au dit polypeptide et qu'on façonne ce mélange sous une forme pharmaceutiquement acceptable de manière à réaliser un médicament à effet retard dans l'organisme, ce dernier étant progressivement libéré à son lieu de destination consécutivement à la biodégradation du polymère porteur.

3. Procédé suivant la revendication 2, caractérisé par le fait qu'on mélange au polymère un polyalcoylène glycol de façon à le plastifier, qu'on y ajoute ledit médicament, puis qu'on moule par la chaleur le produit thermoplastique ainsi obtenu sous forme de granulés, bâtonnets, capsules ou autres particules aptes à être administrées par toutes voies habituelles.

4. Procédé suivant la revendication 1, caractérisé par le fait qu'on le moule pour fabriquer des implants et prothèses biodédradables utilisables en chirurgie.

5. Procédé suivant la revendication 1, caractérisé par le fait que R est choisi parmi les restes méthyle, éthyle, isopropyle, isobutyle, tert-butyle, néopentyle, phényle, benzyle, les alcoyles gras en $C_{10}$ à $C_{22}$ et le polyoxyéthylène glycol méthoxylé comportant de 1 à 100 unités éthylèneoxy.

6. Procédé suivant la revendication 1, caractérisé par le fait que, R et $R^1$ étant liés l'un à l'autre, le maillon formé par ceux-ci est choisi parmi les formules suivantes: $—CH_2—CH_2—$; —CH=CH—; $—CH(CH_3)—CH(CH_3)$; $—C(CH_3=C(CH_3)—$; 1,2-phénylène; cyclohexénylène; cyclopenténylène; cyclopentadiénylène; triméthylène;

$—CH=CH—CH_2—$; 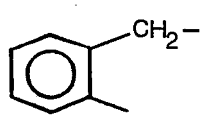

7. Procédé suivant la revendication 1, caractérisé par le fait que la composé (I) est choisi parmi les glutamate et aspartate d'alcoyloxycarbonyl- et aryloxycarbonylméthyle et que le composé II est choisi parmi les copolymères de glutamate ou aspartate d'alcoyloxycarbonyl- et d'aryloxycarbonylméthyle avec un ou plusieurs autres acides aminés choisis parmi l'alanine, la leucine, la valine et la phénylalanine.

8. Procédé suivant la revendication 1, caractérisé par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

9. Procédé suivant la revendication 7, caractérisé par le fait que les groupes alcoyle sont choisis parmi les groupes méthyle, éthyle, propyle, isopropyle, butyle, isobutyle, néopentyle, benzyle, phényle, lauryle, cétyle, oléyle stéaryle.

10. Procédé suivant la revendication 1, caractérisé par le fait qu'on convertit ensuite ledit polypeptide en un film de matière polymérique filmogène par dissolution de celui-ci dans un solvant organique hydrosoluble, on applique une couche de cette solution sur une surface à revêtir, puis on traite cette

surface ainsi revêtue par de l'eau, celle-ci ayant pour effet de dissoudre et d'éliminer le solvant hydrosoluble de manière à former le film de matière filmogène.

11. Procédé suivant la revendication 10, caractérisé par le fait que le solvant est un polyalcoylène glycol, par exemple le polyéthylène glycol (PEG).

12. Procédé suivant la revendication 10, caractérisé par le fait que le solvant est au moins l'un des solvants suivants: PEG, diméthylformamide (DMF), acétone, méthyléthylcétone (MEK), butanol, éthanol, dioxanne, tetrahydrofuranne (THF).

13. Procédé suivant la revendication 10, caractérisé par le fait que ladite solution contient, en outre, un agent porogène.

14. Procédé suivant la revendication 13, caractérisé par le fait que cet agent porogène est le sel moulu très fin, la dissolution des grains de celui-ci dans l'eau de traitement donnant lieu à la formation d'une structure de mousse poreuse.

15. Procédé suivant la revendication 13, caractérisé par le fait que cet agent porogène est un solvant organique volatil et qu'on soumet la couche de revêtement à une lyophilisation de manière que, par évaporation du solvant volatil, il se forme dans la masse une structure poreuse.

16. Procédé suivant la revendication 10, caractérisé par le fait qu'on mélange un médicament à la solution organique, ledit film agissant ensuite comme réservoir de médicament à effet retard dans l'organisme, ce dernier étant progressivement libéré à son lieu de destination consécutivement à la biodégradation du film de polymère porteur.

**Patentansprüche für die Vertragsstaaten: BE CH DE FR GB IT LI LU NL SE**

1. Biologisch abbaubares und in Wasser unlösliches Polypeptid, bestehend aus Alkyloxycarbonyl methyl- oder Aryloxycarbonylmethylpolyaspartat oder -polyglutamate de Formel

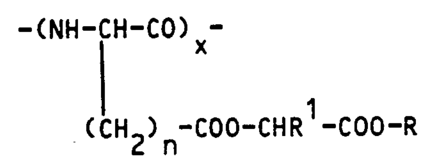

(I)

in der $R^1$ ein niederes Alkyl oder Wasserstoff ist, R ein aliphatischer Rest, insbesondere ein hydroxylierter oder alkoxylierter Polyoxyalkylenrest (linear oder verzweigt), oder ein aromatischer Rest, substituiert oder nicht, ist, oder R und $R^1$ sind untereinander unter Bildung einer Kohlenwasserstoffbrücke verbunden, substituiert oder nicht, mit zwei oder drei Kettengliedern, oder seine Copolymere mit Aminsäuren der Formel

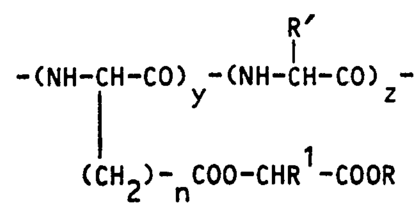

(II)

wo die Gruppen R und $R^1$ die obengenannte Bedeutung haben und wo R' ein nichtcarboxylierter oder carboxylierter Aminosäurerest ist, dessen —COOH frei oder teilweise oder vollständig verestert ist, n den Wert 1 oder 2 hat und x gleich y + z ist und so gewählt ist, daß die Molekularmasse des Polypeptides nicht unter 5000 D liegt.

2. Polypeptid nach Anspruch 1, dadurch gekennzeichnet, daß R aus dem Rest von Methyl, Ethyl, Isopropyl, Isobutyl, tert-Butyl, Neopentyl, Phenyl, Benzyl, den Alkylen mit $C_{10}$ bis $C_{22}$ und dem methoxylierten Polyoxyethylenglykol mit 1 bis 100 Einheiten Ethylenoxy gewählt ist.

3. Polypeptid nach Anspruch 1, dadurch gekennzeichnet, daß bei den miteinander verbundenen R und $R^1$ die Kettenglieder unter den folgenden Formeln ausgewählt werden: $—CH_2—CH_2—$; —CH=CH—; $—CH(CH_3)—CH(CH_3)$; $—C(CH_3)=C(CH_3)—$; 1,2-Phenylen; Cyclohexenylen; Cyclopentenylen, Cyclopentadienylen; Trimethylen; $—CH=CH—CH_2—$;

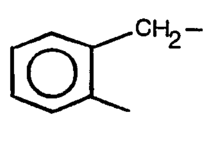

4. Polypeptid nach Anspruch 1, dadurch gekennzeichnet, daß die Verbindung (I) aus dem Glutamat und Aspartat von Alkoxycarbonyl- und Aryloxycarbonylmethyl ausgewählt ist, daß die Verbindung (II) aus den Copolymeren des Glutamats oder Aspartats von Alkyloxycarbonyl- und Aryloxycarbonylmethyl mit einer

oder mehreren anderen Aminosäuren ausgewählt ist, die aus Alanin, Leucin, Valin und Phenylalanin ausgewählt sind.

5. Polypeptid nach Anspruch 1, dadurch gekennzeichnet, daß das Copolymer II aus den Coplymeren des Polyglutamats oder- Aspartats I mit jeweils Glutaminsäure und/oder den Glutamaten der niederen Alkyle und der Asparatinsäure und/oder den Aspartaten niederer Alkyle ausgewählt sind.

6. Polypeptid nach Anspruch 4, dadurch gekennzeichnet, daß die Alkyl- oder Arylgruppen R aus den Gruppen von Methyl, Ethyl, Propyl, Isopropyl, Butyl, Isobutyl, Neopentyl, Benzyl, Phenyl, Lauryl, Cetyl, Oleyl und Stearyl ausgewählt sind.

7. Verwendung des Polypeptides I und seiner Copolymere II nach Anspruch 1 als Medikamentenspeicher mit verzögerter Wirkung im Organismus, wobei letzteres schrittweise an seinen Bestimmungsort infolge des Bioabbaues des Trägerpolymers freigesetzt wird.

8. Verwendung nach Anspruch 7, dadurch gekennzeichnet, daß man das Medikament und das Polymer in homogener Weise mischt und diese Mischung in pharmazeutisch annehmbarer Form ausbildet.

9. Verwendung nach Anspruch 8, dadurch gekennzeichnet, daß man mit dem Polymer ein Polyalkylenglykol derart vermischt, daß es plastifiziert wird, daß man dort ein Medikament einbringt, dann durch Wärme das so erhaltene thermoplastische Produkt in Form von Körnchen, Stäbchen, Kapseln oder anderen Teilchen formt, die auf übliche Art gehandhabt werden.

10. Verwendung der Polymere I und Copolymere II nach Anspruch 1 zur Herstellung biologisch abbaubarer Implantate und Prothesen, die in der Chirurgie verwendbar sind.

11. Verwendung zur Herstellung eines biologisch abbaubaren und in Wasser unlösbaren Polypeptides, bestehend aus Alkyloxycarbonylmethyl- oder Aryloxycarbonylmethyl-polyaspartat oder -polyglutamat oder Formel

$$\begin{array}{l} -(NH-\underset{|}{C}H-CO)_x- \\ \quad\;\; (CH_2)_n-COO-CHR^1-COO-R \end{array} \qquad (I)$$

in der $R^1$ ein niederes Alkyl oder Wasserstoff ist, R ein aliphatischer Rest, insbesondere ein hydroxylierter oder alkoxylierter Polyoxyalkylenrest (linear oder verzweigt), oder ein aromatischer Rest, substituiert oder nicht, ist, oder R und $R^1$ sind untereinander unter Bildung einer Kohlenwasserstoffbrücke verbunden, substituiert oder nicht, mit zwei oder drei Kettengliedern, oder seine Copolymere mit Aminsäuren der Formel

$$\begin{array}{l} \qquad\qquad\qquad\qquad\quad R' \\ -(NH-\underset{|}{C}H-CO)_y-(NH-\overset{|}{C}H-CO)_z- \\ \quad\;\; (CH_2)-_n COO-CHR^1-COOR \end{array} \qquad (II)$$

wo die Gruppen R und $R^1$ die obengenannte Bedeutung haben und wo R′ ein nichtcarboxylierter oder carboxylierter Aminosäurerest ist, dessen —COOH frei oder teilweise oder vollständig verestert ist, n den Wert 1 oder 2 hat und x gleich y + z ist und so gewählt ist, daß die Molekularmasse des Polypeptides nicht unter 5000 D liegt, dadurch gekennzeichnet, daß man die entsprechende Polyaminosäure oder die entsprechende Co-polyaminosäure in Form ihres Salzes mit einem tertiären Amin mit dem Halogenmethylester

$$X—CHR^1—COO—R \qquad (III)$$

reagieren

läßt, wo Y = Cl, Br oder J und R und $R^1$ den erwähnten Definitionen entsprechen, wobei sich die Verbindung I oder II mit gleichzeitiger Beseitigung des Aminsalzes der entsprechenden hydrohalogenierten Säure bildet.

12. Polymeres, filmbildendes Material als Verbandstoff, dadurch gekennzeichnet, daß es aus einer Lösung in einem wasserlöslichen, organischen Lösungsmittel eines Polypeptides besteht, das durch ein biologisch abbaubares und in Wasser unlösliches Alkoxycarbonylmethyl- oder Aryloxycarbonylmethylaspartat oder — polyglutamat der Formel

$$\begin{array}{l} -(NH-\underset{|}{C}H-CO)_x- \\ \quad\;\; (CH_2)_n-COO-CHR^1-COO-R \end{array} \qquad (I)$$

besteht, in der $R^1$ ein niederes Alkyl oder Wasserstoff ist, R ein aliphatischer Rest, insbesondere ein hydroxylierter oder alkoxylierter Polyoxyalkylenrest (linear oder verzweigt), oder ein aromatischer Rest, substituiert oder nicht, ist, oder R und $R^1$ sind untereinander unter Bildung einer Kohlenwasserstoffbrücke verbunden, substituiert oder nicht, mit zwei oder drei Kettengliedern, oder seine Copolymere mit Aminsäuren der Formel

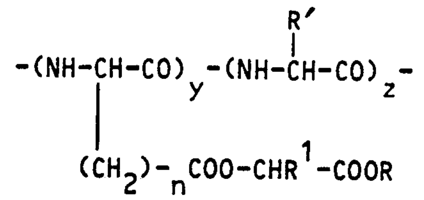

(II)

wo die Gruppen R und $R^1$ die obengenannte Bedeutung haben und wo R′ ein nichtcarboxylierter oder carboxylierter Aminosäurerest ist, dessen —COOH frei oder teilweise oder vollständige verestert ist, n den Wert 1 oder 2 hat und x gleich y + z ist und so gewählt ist, daß die Molekularmasse des Polypeptides nicht unter 5000 D liegt.

13. Polymeres, filmbildenes Material gemäß Anspruch 12, dadurch gekennzeichnet, daß R aus den Resten von Methyl, Ethyl, Isopropyl, Isobutyl, Neopentyl, tert-Butyl, Phenyl, Benzyl und den Alkylen mit $C_{10}$—$C_{22}$ ausgewählt wird.

14. Polymeres, filmbildendes Material nach Anspruch 12, dadurch gekennzeichnet, daß bei den miteinander verbundenen R und $R^1$ ihre Verbindung aus den folgenden Formeln ausgewählt wird: $—CH_2—CH_2—$; —CH=CH—; $—CH(CH_3)—CH(CH_3)$; $—C(CH_3=C(CH_3)$; 1,2-Phenylen, Cyclohexenylen, Cyclopentenylen, Cyclopentadienylen, $—(CH_2)_3—$; $—CH=CH—CH_2$;

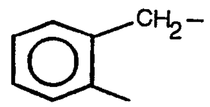

15. Polymeres, filmbildendes Material nach Anspruch 12, dadurch gekennzeichnet, daß die Verbindung I aus dem Glutamat und Aspartat von Alkoxycarbonyl- und Aryloxycarbonylmethyl ausgewählt ist, daß die Verbindung II aus den Copolymeren des Glutamates oder Aspartats von Alkyloxycarbonyl- und Aryloxycarbonylmethyl mit einer oder mehreren anderen Aminosäuren ausgewählt ist, die aus Alanin, Leucin, Valin und Phenylalanin ausgewählt sind.

16. Polymeres, filmbildendes Material nach Anspruch 12, dadurch gekennzeichnet, daß das Copolymer II aus den Copolymeren des Polyglutamats oder Aspartats I mit jeweils Glutaminsäure und/oder den Glutamaten der niederen Alkyle und der Aspartinsäure und/oder den Aspartaten niederer Alkyle ausgewählt sind.

17. Polymeres, filmbildendes Material nach Anspruch 15, dadurch gekennzeichnet, daß die Alkyl- oder Arylgruppen R aus den Gruppen von Methyl, Ethyl, Propyl, Isopropyl, Butyl, Isobutyl, Neopentyl, Benzyl, Phenyl, Lauryl, Cetyl, Oleyl und Stearyl ausgewählt sind.

18. Verwendung des polymeren, filmbildenden Materials nach Anspruch 12, dadurch gekennzeichnet, daß man eine Schicht dieser Lösung auf einer abzudeckenden Oberfläche aufbringt, dann die so bedeckte Oberfläche mit Wasser behandelt, wobei dieses das wasserlösliche Lösungsmittel löst und beseitigt, so daß der Film aus filmbildendem Material geformt wird.

19. Verwendung des polymeren, filmbildenden Materials nach Anspruch 12 als Medikamentenspeicher mit verzögerter Wirkung im Organismus, wobei letzteres schrittweise an seinen Bestimmungsort je nach dem biologischen Abbau des Filmes des Polymerträgers freigesetzt wird.

20. Material nach Anspruch 12, dadurch gekennzeichnet, daß das Lösungsmittel ein Polyalkylenglykol, z.B. das Polyethylenglykol (PEG), ist.

21. Material nach Anspruch 12, dadurch gekennzeichnet, daß das Lösungsmittel wenigstens eines der folgenden Lösungsmittel ist: PEG, Dimethylformamid (DMF), Aceton, Methylethylketon (MEK), Butanol, Ethanol, Dioxan, Tetrahydrofuran (THF).

22. Material nach Anspruch 12, dadurch gekennzeichnet, daß die Lösung überdies ein porenbildendes Mittel enthält.

23. Material nach Anspruch 22, dadurch gekennzeichnet, daß dieses porenbildende Mittel ein sehr fein gemahlenes Salz ist, wobei dessen Lösung der Körnchen in Wasser die Bildung einer porösen Schaumstruktur bewirkt.

24. Material nach Anspruch 22, dadurch gekennzeichnet, daß das porenbildende Mittel ein flüchtiges, organisches Lösungsmittel ist und daß man die Abdeckschicht einer derartigen Lyophilisation unterwirft, das durch Verdampfen des flüchtigen Lösungsmittels in der Masse eine poröse Struktur bildet.

**Patentansprüche für den Vertragsstaat: AT**

1. Verfahren zur Herstellung eines biologisch abbaubaren und in Wasser unlösbaren Polypeptides, bestehend aus Alkyloxycarbonylmethyl- oder Aryloxycarbonylmethyl-polyaspartat oder -polyglutamat der Formel

$$-(NH-\underset{\displaystyle (CH_2)_n-COO-CHR^1-COO-R}{\underset{|}{CH}}-CO)_x- \qquad (I)$$

in der $R^1$ ein niederes Alkyl oder Wasserstoff ist, R ein aliphatischer Rest, insbesondere ein hydroxylierter oder alkoxylierter Polyoxyalkylenrest (linear oder verzweigt), oder ein aromatischer Rest, substituiert oder nicht, ist, oder R und $R^1$ sind untereinander unter Bildung einer Kohlenwasserstoffbrücke verbunden, substituiert oder nicht, mit zwei oder drei Kettengliedern, oder seine Copolymere mit Aminsäuren der Formel

$$-(NH-\underset{\displaystyle (CH_2)-_nCOO-CHR^1-COOR}{\underset{|}{CH}}-CO)_y-(NH-\overset{\displaystyle R'}{\overset{|}{CH}}-CO)_z- \qquad (II)$$

wo die Gruppen R und $R^1$ die obengenannte Bedeutung haben und wo R′ ein nichtcarboxylierter oder carboxylierter Aminosäurerest ist, dessen —COOH frei oder teilweise oder vollständig verestert ist, n den Wert 1 oder 2 hat und x gleich y + z ist und so gewählt ist, daß die Molekularmasse des Polypeptides nicht unter 5000 D liegt, dadurch gekennzeichnet, daß man die entsprechende Polyaminosäure oder die entsprechende Co-polyaminosäure in Form ihres Salzes mit einem tertiären Amin mit dem Halogenmethylester

$$X—CHR^1—COO—R \qquad (III)$$

reagieren Läßt, wo Y = Cl, Br oder J und R und $R^1$ den erwähnten Definitionen entsprechen, wobei sich die Verbindung I oder II mit gleichzeitiger Beseitigung des Aminsalzes der entsprechenden hydrohalogenierten Säure bildet.

2. Verfahren nach Anspruch 1, dadurch gekennzeichnet, daß man dem Polypeptid ein Medikament zumischt und daß man diese Mischung in eine annehmbare, pharmazeutische Form bringt, derart daß ein Medikament mit verzögerter Wirkung im Organismus entsteht, wobei letzteres schrittweise am Bestimmungsort infolge des biologischen Abbaues des Polymerträgers freigesetzt wird.

3. Verfahren nach Anspruch 2, dadurch gekennzeichnet, daß man mit dem Polymer ein Polyalkylenglykol derart vermischt, daß es plastifiziert wird, daß man dort ein Medikament einbringt, dann durch Wärme das so erhaltene thermoplastische Produkt in Form von Körnchen, Stäbchen, Kapseln oder anderen Teilchen formt, die auf übliche Art gehandhabt werden.

4. Verfahren nach Anspruch 1, dadurch gekennzeichnet, daß man es für die Herstellung biologisch abbaubarer Implantate und Prothesen, die in der Chirurgie verwendbar sind, formt.

5. Verfahren nach Anspruch 1, dadurch gekennzeichnet, daß R aus dem Rest von Methyl, Ethyl, Isopropyl, Isobutyl, tert-Butyl, Neopentyl, Phenyl, Benzyl, den Alkylen mit $C_{10}$ bis $C_{22}$ und dem methoxylierten Polyoxyethylenglykol mit 1 bis 100 Einheiten Ethylenoxy gewählt ist.

6. Verfahren nach Anspruch 1, dadurch gekennzeichnet, daß bei den miteinander verbundenen R und $R^1$ die Kettenglieder unter den folgenden Formeln ausgewählt werden: $—CH_2—CH_2—$; $—CH=CH—$; $—CH(CH_3)—CH(CH_3)$; $—C(CH_3=C(CH_3)$; 1,2-Phenylen, Cyclohexenylen, Cyclopentenylen, Cyclopentadienylen, Trimethylen; $—CH=CH—CH_2—$;

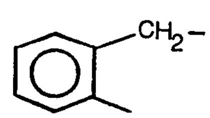

7. Verfahren nach Anspruch 1, dadurch gekennzeichnet, daß die Verbindung I aus dem Glutamat und Aspartat von Alkoxycarbonyl- und Aryloxycarbonylmethyl ausgewählt ist, daß die Verbindung II aus den Copolymeren des Glutamats oder Aspartats von Alkyloxycarbonyl- und Aryloxycarbonylmethyl mit einer

oder mehreren anderen Aminosäuren ausgewählt ist, die aus Alanin, Leucin, Valin und Phenylalanin ausgewählt sind.

8. Verfahren nach Anspruch 1, dadurch gekennzeichnet, daß das Copolymer II aus den Copolymeren des Polyglutamats oder Aspartats I mit jeweils Glutaminsäure und/oder den Glutamaten der niederen Alkyle und der Aspartinsäure und/oder den Aspartaten niederer Alkyle ausgewählt sind.

9. Verfahren nach Anspruch 7, dadurch gekennzeichnet, daß die Alkyl- oder Arylgruppen R aus den Gruppen von Methyl, Ethyl, Propyl, Isopropyl, Butyl, Isobutyl, Neopentyl, Benzyl, Phenyl, Lauryl, Cetyl, Oleyl und Stearyl ausgewählt sind.

10. Verfahren nach Anspruch 1, dadurch gekennzeichnet, daß man schließlich das Polypeptid in einen Film aus filmbildendem Polymermaterial durch Lösung in einem wasserlöslichen, organischen Lösungsmittel umwandelt, daß man eine Schicht dieser Lösung auf einer abzudeckenden Oberfläche aufbringt, dann die so bedeckte Oberfläche mit Wasser behandelt, wobei dieses das wasserlösliche Lösungsmittel löst und beseitigt, so daß der Film aus filmbildendem Material geformt wird.

11. Verfahren nach Anspruch 10, dadurch gekennzeichnet, daß das Lösungsmittel ein Polyalkylenglykol, z.B. Polyethylenglykol (PEG), ist.

12. Verfahren nach Anspruch 10, dadurch gekennzeichnet, daß das Lösungsmittel wenigstens eines der folgenden Lösungsmittel ist: PEG, Dimethylformamid (DMF), Aceton, Methylethylketon (MEDK), Butanol, Ethanol, Dioxan, Tetrahydrofuran (THF).

13. Verfahren nach Anspruch 10, dadurch gekennzeichnet, daß die Lösung überdies ein porenbildendes Mittel enthält.

14. Verfahren nach Anspruch 13, dadurch gekennzeichnet, daß dieses porenbildende Mittel ein sehr fein gemahlenes Salz ist, wobei dessen Lösung der Körnchen in Wasser die Bildung einer porösen Schaumstruktur bewirkt.

15. Verfahren nach Anspruch 13, dadurch gekennzeichnet, daß das porenbildende Mittel ein flüchtiges, organisches Lösungsmittel ist und daß man die Abdeckschicht einer derartigen Lyophilisation unterwirft, das durch Verdampfen des flüchtigen Lösungsmittels in der Masse eine poröse Struktur bildet.

16. Verfahren nach Anspruch 10, dadurch gekennzeichnet, daß man ein Medikament einer organischen Lösung zumischt, wobei dieser Film dann als Medikamentenspeicher mit verzögertem Effekt im Organismus wirkt, wobei letzteres schrittweise an seinen Bestimmungsort infolge des biologischen Abbaues des Filmes des Trägerpolymers freigesetzt wird.

**Claims for the Contracting States: BE CH DE FR GB IT LI LU NL SE**

1. A polypeptide composed of biodegradable and water-insoluble alkyloxycarbonylmethyl or aryloxycarbonylmethyl polyaspartate or polyglutamate corresponding to the formula

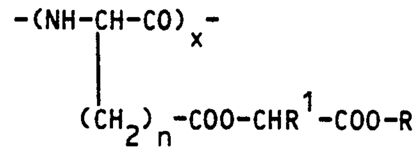

(I)

in which $R^1$ is a lower alkyl or hydrogen, R being a substituted or unsubstituted aliphatic radical, in particular a hydroxylated or alkoxylated (linear or branched) polyoxyalkylene radical, or an aromatic radical optionally substituted; or R and $R^1$ are bound to one another to form a hydrocarbon bridge of two or three links which may or may not be substituted; or its copolymers with amino acids corresponding to the formula

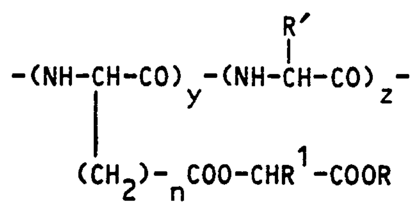

(II)

wherein the groups R and $R^1$ have the meanings given above and wherein R′ is an uncarboxylated or carboxylated amino acid radical of which the —COOH is free or partially or completely esterified; n is 1 or 2 and x which is equal to y + z is selected so that the molecular weight of the polypeptide is not less than 5000 D.

2. A polypeptide according to Claim 1, characterised in that R is selected from the methyl, ethyl, isopropyl, isobutyl, tert.butyl, neopentyl, phenyl, benzil radicals, the $C_{10}$ to $C_{22}$ fatty alkyls and methoxylated polyethylene glycol containing from 1 to 100 oxyethylene units.

3. A polypeptide according to Claim 1, characterised in that, when R and $R^1$ are bound to one another, the link formed by them is selected from the following formulae: $—CH_2—CH_2—$; $—CH=CH—$;

$-CH(CH_3)-CH(CH_3)$; $-C(CH_3)=C(CH_3)-$; 1,2-phenylene; cyclohexenylene; cyclopentenylene, cyclopentadienylene, trimethylene; $-CH=CH-CH_2-$;

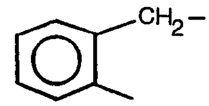

4. A polypeptide according to Claim 1, characterised in that the compound I is selected from alkyloxycarbonylmethyl and aryloxycarbonylmethyl glutamate and aspartate, that compound II is selected from the copolymers of alkyloxycarbonylmethyl and aryloxycarbonylmethyl glutamate or aspartate with one or more other amino acids selected from alanine, leucine, valine and phenylalanine.

5. A polypeptide according to Claim 1, characterised in that the copolymer II is selected from the copolymers of polyglutamate or aspartate I with glutamic acid and/or the lower alkyl glutamates and aspartic acid and/or the lower alkyl aspartates respectively.

6. A polypeptide according to Claim 4, characterised in that the alkyl or aryl groups R are selected from the methyl, ethyl, propyl, isopropyl, butyl, isobutyl, neopentyl, benzyl, phenyl, lauryl, cetyl, oleyl and stearyl groups.

7. Use of the polypeptide I and copolymers thereof II according to Claim 1 as reservoir of a drug having a slow release effect in the organism, the drug being progressively released when at its target place as a result of biodegradation of the polymer carrier.

8. Use according to Claim 7, characterised in that said drug and said polymer are mixed homogeneously and this mixture is shaped into a pharmaceutically acceptable form.

9. Use according to Claim 8, characterised in that a polyalkylene glycol is mixed with the polymer so as to plasticize it, a drug is added thereto, then the thermoplastic product thus obtained is moulded under heat so as to form granulates, rods, capsules or other particles capable of being administered by any conventional methods.

10. Use of polymers I and copolymers II according to Claim 1 for producing biodegradable implants and prostheses which can be used in surgery.

11. A method for the preparation of a polypeptide composed of biodegradable and water-insoluble alkyloxycarbonylmethyl or aryloxycarbonylmethyl polyaspartate or polyglutamate corresponding to the formula

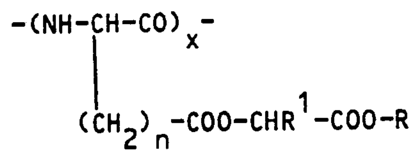

(I)

in which $R^1$ is a lower alkyl or hydrogen, R being a substituted or unsubstituted aliphatic radical, in particular a hydroxylated or alkoxylated (linear or branched) polyoxyalkylene radical, or an aromatic radical optionally substituted; or R and $R^1$ are bound to one another to form a hydrocarbon bridge of two or three links which may or may not be substituted; or its copolymers with amino acids corresponding to the formula

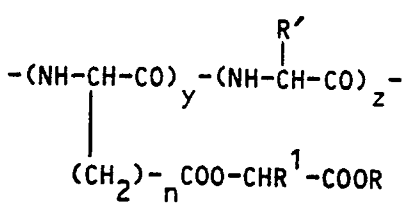

(II)

wherein the groups R and $R^1$ have the meanings given above and wherein R′ is an uncarboxylated or carboxylated amino acid radical of which the —COOH is free or partially or completely esterified; n is 1 or 2 and x which is equal to y + z is selected so that the molecular weight of the polypeptide is not less than 5000 D, characterised in that the corresponding polyamino or copolyamino acid is reacted in the form of its salt of a tertiary amine with a halogenomethyl ester

$$X-CHR^1-COO-R \qquad (III)$$

wherein X = Cl, Br or I and R and $R^1$ correspond to the definitions given above, compound I being formed with simultaneous elimination of the amine salt of the corresponding hydrogen halide.

12. A film-forming polymeric substance which can be used as a dressing, characterised in that it

consists of a solution in a water-soluble organic solvent of a polypeptide composed of biodegradable and water-insoluble alkyloxycarbonylmethyl or aryloxycarbonylmethyl polyaspartate or polyglutamate corresponding to the formula

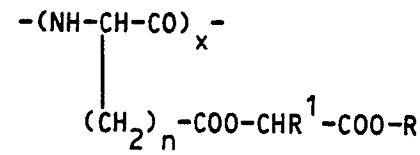

(I)

in which $R^1$ is a lower alkyl or hydrogen, R being a substituted or unsubstituted aliphatic or aromatic radical; or R and $R^1$ are bound to one another so as to constitute a bridge having two or three links of carbon atoms which may or may not be substituted; or the copolymers thereof with other amino acids corresponding to the formula

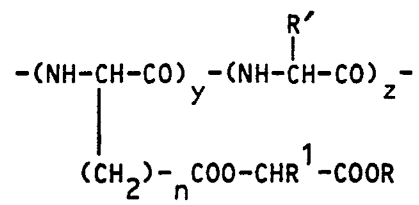

(II)

wherein the groups R and $R^1$ have the meaning given above and wherein R′ is a free or partially or totally esterified amino acid radical, n is 1 or 2 and x which is equal to y + z is selected, so that the molecular weight of the polypeptide is not less than 5000 D.

13. A film-forming polymeric substance according to Claim 12, characterised in that R is selected from the methyl, ethyl, isopropyl, isobutyl, neopentyl, tert.butyl, phenyl, benzyl, radicals and the $C_{10}$ to $C_{22}$ fatty alkyls.

14. A film-forming polymeric substance according to Claim 12, characterised in that, when R and $R^1$ are bound to one another, the bond formed by them is selected from the following formulae: $—CH_2—CH_2—$; $—CH=CH—$; $—CH(CH_3)—CH(CH_3)$; $—C(CH_3)=(CH_3)—$; 1,2-phenylene; cyclohexenylene, cyclopentenylene, cyclopentadienylene; $—(CH_2)_3—$; $—CH=CH—CH_2$;

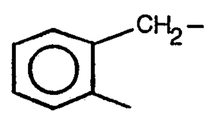

15. A film-forming polymeric substance according to Claim 12, characterised in that the compound (I), is selected from alkyloxycarbonylmethyl and aryloxycarbonylmethyl glutamate and aspartate and compound (II) is selected from the copolymers of alkyloxycarbonylmethyl and aryloxycarbonylmethyl glutamate or aspartate with one or more other amino acids selected from alanine, leucine, valine and phenylalanine.

16. A film-forming polymeric substance according to Claim 12, characterised in that the copolymer II is selected from the copolymers of polyglutamate or aspartate I with glutamic acid and/or the lower alkyl glutamates and respectively with aspartic acid and/or the lower alkyl aspartates.

17. A film-forming polymeric substance according to Claim 15, characterised in that the alkyl groups are selected from the methyl, ethyl, propyl, isopropyl, butyl, isobutyl, tert.butyl, neopentyl, benzyl, phenyl, lauryl, cetyl, stearyl and oleyl groups.

18. Use of the film-forming polymeric substance according to Claim 13, characterised in that a layer of this solution is applied to a surface to be coated, then the coated surface is treated with water, the water having the effect of dissolving and eliminating the water-soluble solvent so as to form the film of film-forming substance.

19. Use of the film-forming polymeric substance according to Claim 12 as a drug reservoir having a retarding effect in the organism, the drug being gradually released to its target as the result of biodegradation of the film of polymer carrier.

20. A substance according to Claim 12, characterised in that the solvent is a polyalkylene glycol, for example polyethylene glycol (PEG).

21. A substance according to Claim 12, characterised in that the solvent is at least one of the following solvents: PEG, dimethylformamide (DMF), acetone, ethylmethylketone (MEK), butanol, ethanol, dioxane, tetrahydrofuran (THF).

22. A substance according to Claim 12, characterised in that said solution also contains a pore-forming agent.

23. A substance according to Claim 22, characterised in that this pore-forming agent is very finely ground salt, dissolution of the grains thereof in the treatment water leading to the formation of a porous foam structure.

24. A substance according to Claim 22, characterised in that this pore-forming agent is a volatile organic solvent and that the covering layer is freeze-dried so as to form a porous structure in the mass by evaporation of the volatile solvent.

**Claims for the Contracting State: AT**

1. A method for the preparation of a polypeptide composed of biodegradable and water-insoluble alkyloxycarbonylmethyl or aryloxycarbonylmethyl polyaspartate or polyglutamate corresponding to the formula

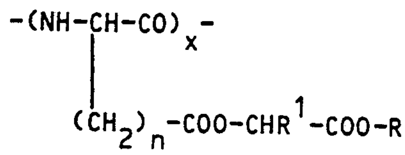
(I)

in which $R^1$ is a lower alkyl or hydrogen, R being a substituted or unsubstituted aliphatic radical, in particular a hydroxylated or alkoxylated (linear or branched) polyoxyalkylene radical, or an aromatic radical optionally substituted; or R and $R^1$ are bound to one another to form a hydrocarbon bridge of two or three links which may or may not be substituted; or its copolymers with amino acids corresponding to the formula

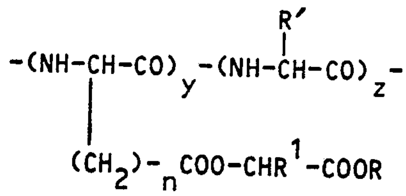
(II)

wherein the groups R and $R^1$ have the meanings given above and wherein R′ is an uncarboxylated or carboxylated amino acid radical of which the —COOH is free or partially or completely esterified; n is 1 or 2 and x which is equal to y + z is selected so that the molecular weight of the polypeptide is not less than 5000 D, characterised in that the corresponding polyamino or copolyamino acid is reacted in the form of its salt of a tertiary amine with a halogenomethyl ester

$$X—CHR^1—COO—R \qquad (III)$$

wherein X = Cl, Br or I and R and $R^1$ correspond to the definitions given above, compound I being formed with simultaneous elimination of the amine salt of the corresponding hydrogen halide.

2. Method according to claim 1, characterised by admixing a drug to said polypeptide and the admixture is shaped into a pharmaceutically acceptable form to achieve a slow release medicament, the drug being progressively released in the organism when at its target place as a result of biodegradation of the polymer carrier.

3. Method according to claim 2, characterised in that a polyalkylene glycol is mixed with the polymer so as to plasticize it, a drug is added thereto, then the thermoplastic product thus obtained is moulded under heat so as to form granulates, rods, capsules or other particles capable of being administered by any conventional methods.

4. Method according to claim 1, characterised in that the polymers are moulded for producing biodegradable implants and prostheses which can be used in surgery.

5. Method according to claim 1, characterised in that R is selected from the methyl, ethyl, isopropyl, isobutyl, tert.butyl, neopentyl, phenyl, benzyl radicals, the $C_{10}$ to $C_{22}$ fatty alkyls and methoxylated polyethylene glycol containing from 1 to 100 oxyethylene units.

6. Method according to claim 1, characterised in that R and $R^1$ being bound to one another, the link formed by them is selected from the following formulae: $—CH_2—CH_2—$; $—CH=CH—$; $—CH(CH_3)—CH(CH_3)$; $—C(CH_3)=C(CH_3)—$; 1,2-phenylene; cyclohexenylene; cyclopentenylene, cyclopentadienylene, trimethylene; $—CH=CH—CH_2—$;

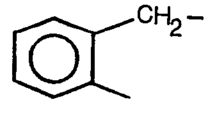

7. Method according to claim 1, characterised in that the compound I is selected from alkyloxycarbonylmethyl and aryloxycarbonylmethyl glutamate or aspartate with one or more other amino acids selected from alanine, leucine, valine and phenylalanine.

8. Method according to claim 1, characterised in that the copolymer II is selected from the copolymers of polyglutamate or aspartate I with glutamic acid and/or the lower alkyl glutamates and aspartic acid and/or the lower alkyl aspartates respectively.

9. Method according to claim 4, characterised in that the alkyl or aryl groups R are selected from the methyl, ethyl, propyl, isopropyl, butyl, isobutyl, neopentyl, benzyl, phenyl, lauryl, cetyl, oleyl and stearyl groups.

10. Method according to claim 1, characterised in that said polypeptide is thereafter converted to a film of film-forming material by dissolving the polypeptide in an organic hydrosoluble solvent, the solution is applied to a surface to be coated, then the coated surface is treated with water, the water having the effect of dissolving and eliminating the water-soluble solvent so as to form the film of film-forming substance.

11. Method according to claim 10, characterised in that the solvent is a polyalkylene glycol, for example polyethylene glycol (PEG).

12. Method according to claim 10, characterised in that the solvent is at least one of the following solvents: PEG, dimethylformamide (DMF), acetone, ethylmethylketone (MEK), butanol, ethanol, dioxane, tetrahydrofuran (THF).

13. Method according to claim 10, characterised in that said solution also contains a pore-forming agent.

14. Method according to claim 13, characterised in that this pore-forming agent is very finely ground salt, dissolution of the grains thereof in the treatment water leading to the formation of a porous foam structure.

15. Method according to claim 13, characterised in that this pore-forming agent is a volatile organic solvent and that the covering layer is freeze-dried so as to form a porous structure in the mass by evaporation of the volatile solvent.

16. Method according to claim 10, characterised in that a drug is admixed with the organic solution, said film acting subsequently as reservoir of the drug having a slow release effect in the organism, the drug being progressively released when at its target place as a result of biodegradation of the polymer carrier.